# EUROPÄISCHE PATENTANMELDUNG

(11) **EP 1 683 499 A1**
(43) Veröffentlichungstag der Anmeldung: **26.07.2006**
(21) Anmeldenummer: 06000134.4
(22) Anmeldetag: 04.01.2006
(51) Int. Cl.: A61C 19/00, A61B 19/00, B24C 1/02

(54) **Reinigungsvorrichtung für Behandlungsinstrumente**

(30) Priorität: 13.01.2005 DE 102005001700
(71) Anmelder: Julius-Maximilians-Universität Würzburg, 97070 Würzburg (DE)
(72) Erfinder: Haas, Manfred, 97264 Helmstadt (DE); Muth, Andreas, 97250 Erlabrunn (DE); Helms, Jan, Prof. Dr., 97080 Würzburg (DE)
(74) Vertreter: Rothkopf, Ferdinand

(57) **Zusammenfassung**

Eine Instrumenten-Reinigungsvorrichtung (10) für mindestens ein kontaminiertes Behandlungsinstrument (12), insbesondere für mehrfach zu verwendende Bohr- oder Fräswerkzeuge und dergleichen, ist erfindungsgemäß dadurch gekennzeichnet, dass das mindestens eine kontaminierte Behandlungsinstrument (12) an der Reinigungsvorrichtung (10) zum Abtragen der Kontamination in einem Partikelstrahl angeordnet ist.

## Beschreibung

### Hintergrund der Erfindung

Die Erfindung betrifft eine Instrumenten-Reinigungsvorrichtung für mindestens ein kontaminiertes Behandlungsinstrument, wie beispielsweise chirurgische Instrumente, insbesondere für mehrfach zu verwendende Bohr- oder Fräswerkzeuge und dergleichen. Ferner betrifft die Erfindung ein Instrumenten-Reinigungsverfahren zum Reinigen mindestens eines derartigen kontaminierten Behandlungsinstruments.

Bei ärztlichen Eingriffen, wie beispielsweise bei hals-nasen-ohrenärztlichen Operationen an der Schädelbasis, ist es häufig notwendig, Knochen zu bearbeiten und zu entfernen. Dies geschieht mit Hilfe chirurgischer Behandlungsinstrumente bzw. Werkzeuge, die im klinischen Sprachgebrauch z.B. als "Fräser" und "Bohrer" bezeichnet werden. Die Behandlungsinstrumente sind Miniatur-Präzisionsinstrumente, die oftmals mit Diamantsplittern besetzt sind und die dadurch sehr scharfkantige Oberflächen aufweisen.

Um bei den chirurgischen Operationen bestmögliche Ergebnisse zu erzielen, ist es notwendig, dass die Behandlungsinstrumente scharf bleiben und die Vertiefungen zwischen den schneidenden Kanten nicht auf Dauer durch biologisches Material, wie beispielsweise Knochen oder Knorpel, zugesetzt sind.

Während eines medizinischen Eingriffs kann es aber durchaus geschehen, dass sich an den Behandlungsinstrumenten durch abgetragenes Material Grate aufbauen oder die diamantierten Oberflächen sich zusetzen. Dies geschieht z.B., wenn bei einem Bohrvorgang nicht ausreichend gespült oder mit zu großem Druck auf die zu bearbeitende Oberfläche eingewirkt wird. Die derart kontaminierten Behandlungsinstrumente können nicht weiter verwendet werden.

Es ist bekannt, die benutzen Behandlungsinstrumente per Hand mit Bürsten zu reinigen, dann in einem Reinigungsbad mit Ultraschall zu behandeln und zuletzt zu sterilisieren. Nach dieser Reinigungsprozedur stehen die Behandlungsinstrumente wieder für weitere Eingriffe bereit.

Wenn es nicht gelingt, die Rückstände bzw. Verschmutzungen an den Behandlungsinstrumenten vollständig zu entfernen, sollten diese nicht wieder verwendet werden. Eine Übertragung potentiell infektiösen Materials von einem Patienten auf einen anderen muss sicher ausgeschlossen werden. Eine Einmalverwendung ist im Routinebetrieb einer Klinik wegen des hohen Preises der Präzisionsinstrumente aber nicht dauerhaft zu finanzieren.

### Zugrunde liegende Aufgabe

Der Erfindung liegt die Aufgabe zugrunde, eine Vorrichtung und ein Verfahren zur Reinigung insbesondere chirurgischer Instrumente bereitzustellen, bei denen die oben genannten Probleme überwunden und eine rückstandsfreie Säuberung benutzter Wirkflächen der Instrumente möglich ist. Die Reinigung soll ferner vergleichsweise kostengünstig durchgeführt werden können.

### Erfindungsgemäße Lösung

Diese Aufgabe ist erfindungsgemäß mit einer gattungsgemäßen Instrumenten-Reinigungsvorrichtung gelöst, bei der das mindestens eine kontaminierte Behandlungsinstrument an der Reinigungsvorrichtung zum Abtragen der Kontamination in einem Partikel-, Sand- oder Pulverstrahl angeordnet ist. Ferner ist die Aufgabe mit einem gattungsgemäßen Instrumenten-Reinigungsverfahren gelöst, bei welchem das mindestens eine kontaminierte Behandlungsinstrument zum Abtragen der Kontamination mit Partikeln bestrahlt wird. Schließlich ist die Aufgabe auch durch eine Verwendung einer Partikelstrahl-Reinigungsvorrichtung zum Reinigen mindestens eines kontaminierten Behandlungsinstruments, insbesondere zum Reinigen von mehrfach zu verwendenden Bohr- oder Fräswerkzeugen oder dergleichen gelöst.

Erfindungsgemäß wird im Hinblick auf die Reinigung von Behandlungsinstrumenten ein völlig neuartiger Weg gewählt. Es wird bei der Reinigung nicht wie bisher mit Bürsten gearbeitet, sondern es wird gezielt auf das zu reinigende Instrument ein Partikelstrahl ausgerichtet, mit dem die an der Oberfläche des Behandlungsinstruments anhaftenden Rückstände abgetragen werden. Auf diese Weise, so hat sich durch entsprechende Nachuntersuchungen gezeigt, können insbesondere chirurgische Instrumente wie Bohrer und Fräser rückstandsfrei gereinigt werden.

Außer für chirurgische Instrumente ist das erfindungsgemäße Reinigungsverfahren vorteilhaft auch für kosmetische Instrumente geeignet, oder zur Reinigung zahnärztlicher Feilen, wie sie beispielsweise zur Entfernung eines abgestorbenen Nervs aus einem Zahnkanal verwendet werden.

Das erfindungsgemäße Vorgehen kann grundsätzlich manuell durchgeführt werden. Erfindungsgemäß bevorzugt ist aber eine automatisierte Reinigung, bei der der genannte Partikelstrahl ganz gezielt über eine bestimmte Zeitdauer hinweg auf einzelne Abschnitte der zu reinigenden Oberflächen gerichtet wird. Auf diese Weise kann erfindungsgemäß die ansonsten bestehende Abhängigkeit von der Qualität der menschlichen Arbeit vollständig ausgeschlossen werden.

Besonders wichtig ist bei der erfindungsgemäßen Lösung, dass diese nicht zu einer Beeinträchtigung der Funktionalität der gereinigten Werkzeuge führt. Man würde wohl zunächst annehmen, dass bei einem scharfkantigen Werkzeug, auf das mit hohem Druck ein Partikel- bzw. Sandstrahl gerichtet wird, auch an seinen Schneidkanten in nicht annehmbarer Weise geschädigt wird. Dies umso mehr, als mit dem Sandstrahl auch besonders hartnäckige Verunreinigungen entfernt werden sollen. Es hat sich bei erfindungsgemäßen Untersuchungen jedoch gezeigt, dass dieses Vorurteil nicht richtig ist und sehr wohl ein Druckbereich und eine Partikelqualität gefunden werden können, bei denen eine Schädigung der Werkzeuge ausgeschlossen ist. Daher werden durch das erfindungsgemäße Vorgehen die Schneidkanten der Werkzeuge auch bei wiederholter Reinigung nicht stumpf.

### Vorteilhafte Weiterbildungen der Erfindung

Bei einer vorteilhaften Weiterbildung der Erfindung ist eine Düse zum Aussprühen des Partikelstrahls vorgesehen, welche insbesondere ortsfest angeordnet ist. Um eine besonders kostengünstige Lösung zu schaffen kann als oben genannte Düse durchaus jene von bekannten Pulver- bzw. Sandstrahlgeräten verwendet werden, mit denen ein Feinpartikelstrahl ausgebracht werden kann. Solche Geräte sind beispielsweise als Pulverstrahlgeräte für supragingivale Zahnoberflächen bekannt. Ferner sind Feinstrahlgeräte bekannt, welche bisher im Werkzeug- und Formenbau, in der Uhrenfabrikation oder der Glas- und Keramikindustrie beispielsweise zum Aufrauen oder Mattieren eingesetzt werden. Die Düse ist dabei vorteilhaft ortsfest angeordnet, weil, wie nachfolgend noch erläutert werden wird, es leichter möglich ist, die Instrumente zu bewegen, als die Düse und deren Zuleitung für das Feinstrahlmaterial.

Da, wie oben erläutert, erfindungsgemäß eine möglichst automatisierte Reinigung der Behandlungsinstrumente angestrebt wird, ist es ferner besonders von Vorteil, wenn an der Reinigungsvorrichtung eine Halteeinrichtung zum Haltern des mindestens einen kontaminierten Behandlungsinstruments vorgesehen ist, mit welcher insbesondere das mindestens eine Behandlungsinstrument, welches in dem Partikelstrahl angeordnet ist, vor der ortsfest angeordneten Düse bewegt werden kann. Mit der Halteeinrichtung kann eine Bewegung des zu reinigenden Instruments im Partikelstrahl wiederholbar vorgegeben und dadurch die gewünschte Reinigungsgüte dauerhaft sichergestellt werden.

Besonders kostengünstig wird eine derartige Lösung, indem die Halteeinrichtung mit einem Schwenkmechanismus gestaltet ist, mit dem das mindestens eine Operationswerkzeug in dem Partikelstrahl geschwenkt werden kann. Durch ein Vorsehen eines Schwenkbereichs von etwa 60° bis 120°, insbesondere von etwa 90°, können an bekannten Behandlungsinstrumenten alle zu reinigenden Abschnitte in der gewünschten Weise dem erfindungsgemäßen Partikelstrahl ausgesetzt werden.

Damit bei dieser Schwenkbewegung zugleich mit einer ortsfesten und daher besonders kostengünstig angebrachten Düse für den Partikelstrahl gearbeitet werden kann, ist die Reinigungsvorrichtung vorteilhaft für Behandlungsinstrumente vorgesehen, welche einen Instrumentenkopf aufweisen, und der Schwenkmechanismus ist derart gestaltet, dass sich der Drehpunkt des mindestens einen Behandlungsinstruments, welches in dem Partikelstrahl angeordnet ist, beim Schwenken im Wesentlichen im Zentrum des Instrumentenkopfes befindet.

Weil die zu reinigenden Behandlungsinstrumente ferner in der Regel rotationssymmetrische Werkzeuge sind, können diese über ihre gesamte Wirkfläche hinweg besonders gleichmäßig und rückstandsfrei gereinigt werden, indem die Halteeinrichtung mit einem Drehmechanismus gestaltet ist, mittels dem das mindestens eine Operationswerkzeug, welches in dem Partikelstrahl angeordnet ist, im Wesentlichen um seine Längsachse gedreht werden kann.

Damit mit der erfindungsgemäßen Vorrichtung insbesondere automatisiert auch eine Mehrzahl Instrumente vergleichsweise schnell gereinigt werden kann, ohne dass dazu ständig ein Überwachen und Umrüsten der Reinigungsvorrichtung notwendig wäre, ist ferner vorteilhaft eine Halteeinrichtung vorgesehen, welche zum gleichzeitigen Haltern mehrerer kontaminierter Behandlungsinstrumente angepasst ist und mit der einzelne Behandlungsinstrumente aufeinander folgend in dem Partikelstrahl angeordnet werden können.

Die Halteeinrichtung ist dabei besonders leicht zu bestücken, indem sie vorteilhaft als eine Art Magazin oder Revolvertrommel gestaltet ist, in welche mehrere Behandlungsinstrumente einsetzbar sind.

Die Revolvertrommel kann insgesamt drehbar und schwenkbar gestaltet sein und mit mehreren Drehbuchsen versehen sein, welche über den Umfang der Trommel verteilt angeordnet sind. In die Drehbuchsen können dann einzelne oder auch Gruppen von Instrumenten eingesetzt werden. Wenn eine derartige Revolvertrommel geschwenkt und zugleich in Drehung versetzt wird, kann ferner auf besonders einfache Weise während der Drehung eine einzelne Drehbuchse zeitweise gebremst werden. Die Drehbuchse dreht sich durch das Bremsen relativ zu der rotierenden Revolvertrommel. Das in der Drehbuchse gehaltene Behandlungsinstrument wird dadurch um einen bestimmten Winkel um seine Längsachse gedreht und gelangt beim nächsten Vorbeilauf bzw. Durchlauf mit einer anderen Drehlage in den Partikelstrahl.

Um Instrumente an der erfindungsgemäßen Halteeinrichtung bzw. der Revolvertrommel einfach bestücken und entnehmen zu können, ist es ferner von Vorteil, wenn die Reinigungsvorrichtung zum Reinigen von Behandlungsinstrumenten vorgesehen ist, welche einen Instrumentenschaft aufweisen, und die Halteeinrichtung derart gestaltet ist, dass dort mindestens ein Behandlungsinstrument mit seinem Instrumentenschaft eingeschoben werden kann. Vor einem Bestücken eines Behandlungsinstruments wird bevorzugt auf dessen Instrumentenschaft ein O-Ring abdichtend aufgeschoben. Der O-Ring kommt beim Einschieben des Instrumentenschafts in die Revolvertrommel bzw. in eine von deren Drehbuchsen an der Halteeinrichtung zur Anlage und wird nachfolgend entlang dem Instrumentenschaft verschoben, bis das Behandlungsinstrument seine Endlage erreicht hat. Der derart angeordnete O-Ring verhindert ein Eindringen von Feinpartikeln in die Aufnahme des Instrumentenschafts.

### Kurzbeschreibung der Zeichnungen

Nachfolgend wird ein Ausführungsbeispiel einer erfindungsgemäßen Instrumenten-Reinigungsvorrichtung anhand der beigefügten schematischen Zeichnungen näher erläutert. Es zeigt:

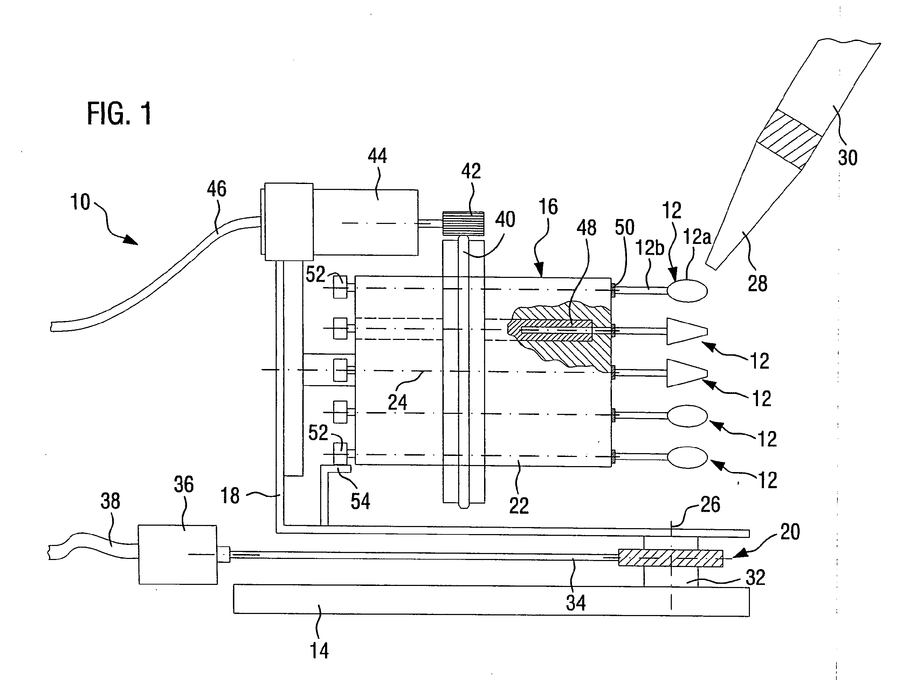

- Fig. 1: eine Seitenansicht eines Ausführungsbeispiels einer erfindungsgemäßen Instrumenten-Reinigungsvorrichtung und

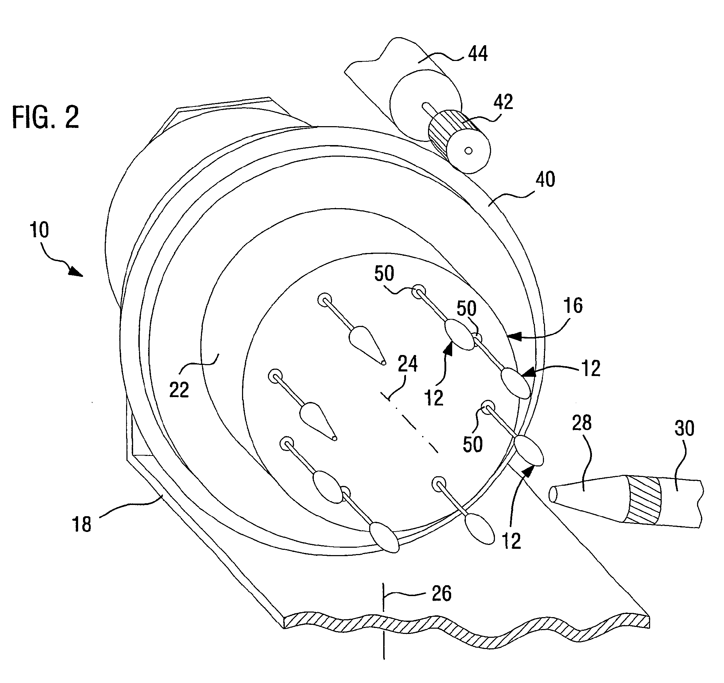

- Fig. 2: eine perspektivische Ansicht des Ausführungsbeispiels gemäß Fig. 1.

### Detaillierte Beschreibung des Ausführungsbeispiels

In den Fig. ist eine Instrumenten-Reinigungsvorrichtung 10 dargestellt, welche zum Reinigen von kontaminierten Behandlungsinstrumenten 12 vorgesehen ist.

Die Instrumenten-Reinigungsvorrichtung 10 ist aus einer Grundplatte 14 und einer darauf angeordneten Halteeinrichtung 16 für die Behandlungsinstrumente 12 aufgebaut. Gebildet ist die Halteeinrichtung 16 aus einem im Wesentlichen L-förmigen Schwenkgestell 18, von dem ein erster, waagrecht liegender Schenkel über ein Schwenkgetriebe 20 auf der Grundplatte 14 befestigt ist; sowie mit einer Revolvertrommel 22, welche an dem zweiten, nach oben ragenden Schenkel des L-förmigen Schwenkgestells 18 angeordnet ist.

Die Revolvertrommel 22 trägt die Behandlungsinstrumente 12 und kann um eine im Wesentlichen waagrecht liegende Drehachse 24 rotiert werden. Zugleich kann das Schwenkgestell 18 um eine im Wesentlichen senkrecht stehende Schwenkachse 26 geschwenkt werden. Auf diese Weise werden die Behandlungsinstrumente 12 vor einer Düse 28 bewegt, welche vor der Revolvertrommel 22 angeordnet ist.

Die Düse 28 ist relativ zur Grundplatte 14 ortsfest angebracht und mit einer Partikelzuführung 30 verbunden, durch die mit einem Druck von zwischen etwas 0,3 bis 8 bar Partikel aus beispielsweise Kunststoff mit einer bevorzugten Korngröße von zwischen etwa 5 bis 500 µm hindurchgefördert werden können.

Die Partikel treten als Partikelstrahl aus der Düse 28 aus und treffen mit derart hoher Geschwindigkeit auf den Instrumentenkopf 12a eines der Behandlungsinstrumente 12 auf, dass von diesem in einem bestimmten Bereich die zu entfernenden Kontaminationen vollständig und rückstandslos abgetragen werden können.

Im Detail ist das Schwenkgetriebe 20 mit einem Schwenkkranz 32 gebildet, welcher zwischen der Grundplatte 14 und dem waagrechten Schenkel des L-förmigen Schwenkgestells 18 angeordnet ist. Der Schwenkkranz 32 legt die Schwenkachse 26 fest und ist derart angeordnet, dass sich diese durch den jeweils zu reinigenden Instrumentenkopf 12a hindurch erstreckt. Es wird auf diese Weise der Instrumentenkopf 12a vor der Düse 28 so geschwenkt, dass beim Schwenken stets der Partikelstrahl auf den Instrumentenkopf 12a gerichtet bleibt.

Angetrieben wird der Schwenkkranz 32 des Schwenkgetriebes 20 durch eine Schneckenwelle 34, welche von einem Schwenkmotor 36 gedreht werden kann. Der Schwenkmotor 36 ist dazu durch eine elektrische Leitung 38 an eine nicht dargestellte Steuerung der Instrumenten-Reinigungsvorrichtung 10 angeschlossen.

Die Revolvertrommel 22 ist an ihrer Mantelfläche mit einem sie umgebenden Reibrad 40 versehen, an dem ein Antriebsrad 42 eines Drehmotors 44 angreift. Der Drehmotor 44 ist ebenfalls über eine elektrische Leitung 46 an der Steuerung der Instrumenten-Reinigungsvorrichtung 10 angeschlossen.

Als eigentliche Aufnahme der Behandlungsinstrumente 12 sind in der Revolvertrommel 22 insgesamt acht Drehbuchsen 48 eingesetzt, welche sich im Wesentlichen parallel zur Drehachse 24 erstrecken und über den Umfang der Revolvertrommel 22 in gleichmäßigen Abständen verteilt sind. In die Drehbuchsen 48 sind jeweils einzeln die Behandlungsinstrumente 12 mit ihrem Instrumentenschaft 12b eingesteckt. Dabei ist auf den Instrumentenschaft 12b ein O-Ring 50 aufgeschoben, welcher an jener Stirnfläche der Revolvertrommel 22 zum Anliegen kommt, welche der Düse 28 gegenüberliegt. Die derart angeordneten O-Ringe 50 verhindern ein Eindringen von Partikeln des Partikelstrahls in die Aufnahmen der Instrumentenschäfte 12b.

An der von der Düse 28 abgewandten Stirnfläche der Revolvertrommel 22 ragen aus dieser Bremsräder 52 heraus, welche jeweils mit einer der Drehbuchsen 48 drehfest verbunden sind. Den Bremsrädern 52 ist eine Bremsfläche 54 zugeordnet, welche an dem Schwenkgestell 18 ortsfest angebracht ist. Die Bremsfläche 54 ist dabei derart gestaltet und angeordnet, dass beim Rotieren der Revolvertrommel 22 die Bremsräder 52 an der Bremsfläche 54 vorbeilaufen und an dieser einzeln gebremst werden. Durch das Bremsen wird das einzelne Bremsrad 54 zusammen mit der zugehörigen Drehbuchse 48 relativ zur Revolvertrommel 22 um einen vorbestimmten Winkel von bevorzugt etwa 45° gedreht. Auf diese Weise wird die Drehlage des sich in der Drehbuchse 48 befindlichen Behandlungsinstruments 12 in Bezug auf den aus der Düse 28 austretenden Partikelstrahl verändert.

Im Zusammenwirken mit der nicht dargestellten Steuerung werden die beiden Motoren 36 und 44 ferner derart angesteuert, dass in der Revolvertrommel 22 bei einer Reinigungsdauer von beispielsweise zirka 3 Minuten ein einzelnes Behandlungsinstrument 12 mehrfach an der Düse 28 vorbeiläuft und zugleich in einem Winkel von etwa 90° geschwenkt wird.

Auf diese Weise werden an den Behandlungsinstrumenten 12 sämtliche zu reinigenden Bereiche der jeweiligen Instrumentenköpfe 12a dem Partikelstrahl ausgesetzt und von dort vorhandenen Kontaminationen oder Rückständen gereinigt.

Abschließend sei angemerkt, dass sämtlichen Merkmalen, die in den Anmeldungsunterlagen und insbesondere in den abhängigen Ansprüchen genannt sind, trotz dem vorgenommenen formalen Rückbezug auf einen oder mehrere bestimmte Ansprüche, auch einzeln oder in beliebiger Kombination eigenständiger Schutz zukommen soll.

### Bezugszeichenliste

- 10: Instrumenten-Reinigungsvorrichtung
- 12: Behandlungsinstrument
- 12a: Instrumentenkopf
- 12b: Instrumentenschaft
- 14: Grundplatte
- 16: Halteeinrichtung
- 18: Schwenkgestell
- 20: Schwenkgetriebe
- 22: Revolvertrommel
- 24: Drehachse
- 26: Schwenkachse
- 28: Düse
- 30: Partikelzuführung
- 32: Schwenkkranz
- 34: Schneckenwelle
- 36: Schwenkmotor
- 38: elektrische Leitung
- 40: Reibrad
- 42: Antriebsrad
- 44: Drehmotor
- 46: elektrische Leitung
- 48: Drehbuchse
- 50: O-Ring
- 52: Bremsrad
- 54: Bremsfläche

## Patentansprüche

1. Instrumenten-Reinigungsvorrichtung (10) für mindestens ein kontaminiertes Behandlungsinstrument (12), insbesondere für mehrfach zu verwendende Bohr-oder Fräswerkzeuge und dergleichen,
**dadurch gekennzeichnet, dass** das mindestens eine kontaminierte Behandlungsinstrument (12) an der Reinigungsvorrichtung (10) zum Abtragen der Kontamination in einem Partikelstrahl angeordnet ist.

2. Instrumenten-Reinigungsvorrichtung nach Anspruch 1,
**dadurch gekennzeichnet, dass** eine Düse (28) zum Aussprühen des Partikelstrahls vorgesehen ist, welche insbesondere ortsfest angeordnet ist.

3. Instrumenten-Reinigungsvorrichtung nach Anspruch 1 oder 2,
**dadurch gekennzeichnet, dass** eine Halteeinrichtung (16) zum Haltern des mindestens einen kontaminierten Behandlungsinstruments (12) vorgesehen ist, mit welcher insbesondere das mindestens eine Behandlungsinstrument (12), welches in dem Partikelstrahl angeordnet ist, vor der ortsfest angeordneten Düse (28) bewegt werden kann.

4. Instrumenten-Reinigungsvorrichtung nach Anspruch 3,
**dadurch gekennzeichnet, dass** die Halteeinrichtung (16) mit einem Schwenkmechanismus (18, 20, 32, 34, 36) gestaltet ist, mit dem das mindestens eine Behandlungsinstrument (12) in dem Partikelstrahl geschwenkt werden kann.

5. Instrumenten-Reinigungsvorrichtung nach Anspruch 4,
**dadurch gekennzeichnet, dass** die Reinigungsvorrichtung (10) für Behandlungsinstrumente (12) vorgesehen ist, welche einen Instrumentenkopf (12a) aufweisen, und der Schwenkmechanismus (18, 20, 32, 34, 36) derart gestaltet ist, dass sich die Schwenkachse (26) des mindestens einen Behandlungsinstruments (12), welches in dem Partikelstrahl angeordnet ist, beim Schwenken im Wesentlichen im Zentrum des Instrumentenkopfes (12a) befindet.

6. Instrumenten-Reinigungsvorrichtung nach einem der Ansprüche 1 bis 5,
**dadurch gekennzeichnet, dass** die Halteeinrichtung (16) mit einem Drehmechanismus (48, 52, 54) gestaltet ist, mittels dem das mindestens eine Behandlungsinstrument (12), welches in dem Partikelstrahl angeordnet ist, im Wesentlichen um seine Längsachse gedreht werden kann.

7. Instrumenten-Reinigungsvorrichtung nach einem der Ansprüche 1 bis 6,
**dadurch gekennzeichnet, dass** eine Halteeinrichtung (18, 20, 32, 34, 36) vorgesehen ist, welche zum gleichzeitigen Haltern mehrerer kontaminierter Behandlungsinstrumente (12) angepasst ist und mit der einzelne Behandlungsinstrumente (12) aufeinander folgend in dem Partikelstrahl angeordnet werden können.

8. Instrumenten-Reinigungsvorrichtung nach Anspruch 7,
**dadurch gekennzeichnet, dass** die Halteeinrichtung (18, 20, 32, 34, 36) mit einer Art Revolvertrommel (22) gestaltet ist, in welche mehrere Behandlungsinstrumente (12) einsetzbar sind.

9. Instrumenten-Reinigungsvorrichtung nach Anspruch 8,
**dadurch gekennzeichnet, dass** die Revolvertrommel (22) insgesamt drehbar und schwenkbar gestaltet ist und mehrere Drehbuchsen (48) über ihren Umfang verteilt angeordnet sind, in denen jeweils ein einzelnes Behandlungsinstrument (12) eingesetzt werden kann.

10. Instrumenten-Reinigungsvorrichtung nach Anspruch 7, 8 oder 9,
**dadurch gekennzeichnet, dass** die Reinigungsvorrichtung (10) zum Reinigen von Behandlungsinstrumenten (12) vorgesehen ist, welche einen Instrumentenschaft (12b) aufweisen, und die Halteeinrichtung (18, 20, 32, 34, 36) derart gestaltet ist, dass dort mindestens ein Behandlungsinstrument (12) mit seinem Instrumentenschaft (12b) eingeschoben werden kann.

11. Instrumenten-Reinigungsverfahren zum Reinigen mindestens eines kontaminierten Behandlungsinstruments (12), insbesondere zum Reinigen von mehrfach zu verwendenden Bohr- oder Fräswerkzeugen und dergleichen,
**dadurch gekennzeichnet, dass** das mindestens eine kontaminierte Behandlungsinstrument (12) zum Abtragen der Kontamination mit Partikeln bestrahlt wird.

12. Instrumenten-Reinigungsverfahren nach Anspruch 11,
**dadurch gekennzeichnet, dass** der Partikelstrahl durch eine insbesondere ortsfest angeordnete Düse (28) ausgesprüht wird.

13. Instrumenten-Reinigungsverfahren nach Anspruch 11 oder 12,
**dadurch gekennzeichnet, dass** das mindestens eine kontaminierte Behandlungsinstrument (12), welches mit Partikeln bestrahlt wird, von einer Halteeinrichtung (16) gehalten und insbesondere vor der ortsfest angeordneten Düse (28) bewegt wird.

14. Instrumenten-Reinigungsverfahren nach Anspruch 13,
**dadurch gekennzeichnet, dass** das mindestens eine Behandlungsinstrument (12) in dem Partikelstrahl geschwenkt wird.

15. Instrumenten-Reinigungsverfahren nach Anspruch 14,
**dadurch gekennzeichnet, dass** die Reinigungsvorrichtung (10) zum Reinigen von Behandlungsinstrumenten (12) vorgesehen ist, welche einen Instrumentenkopf (12a) aufweisen, und das mindestens eine Behandlungsinstrument (12), welches mit Partikeln bestrahlt wird, um einen Drehpunkt geschwenkt wird, der sich im Wesentlichen im Zentrum des Instrumentenkopfes (12a) befindet.

16. Instrumenten-Reinigungsverfahren nach einem der Ansprüche 11 bis 15,
**dadurch gekennzeichnet, dass** das mindestens eine Behandlungsinstruments (12), welches mit Partikeln bestrahlt wird, im Wesentlichen um seine Längsachse gedreht wird.

17. Instrumenten-Reinigungsverfahren nach einem der Ansprüche 11 bis 16,
**dadurch gekennzeichnet, dass** gleichzeitig mehrere kontaminierte Behandlungsinstrumente (12) gehalten und aufeinander folgend mit Partikeln bestrahlt werden.

18. Verwendung einer Partikelstrahl-Reinigungsvorrichtung (10) zum Reinigen mindestens eines kontaminierten Behandlungsinstruments (12), insbesondere zum Reinigen von mehrfach zu verwendenden Bohr- oder Fräswerkzeugen und dergleichen.
